# EUROPEAN PATENT APPLICATION

(11) **EP 0 869 114 A1**
(43) Date of publication of application: **07.10.1998**
(21) Application number: 97105620.5
(22) Date of filing: 04.04.1997
(51) Int. Cl.: C07C 219/06, C07C 213/06, C11D 1/46, C11D 1/62, D21H 17/14, B01D 19/04

(54) **Composition useful for softening applications and processes for the preparation thereof**

(71) Applicant: Dow Europe S.A., 8810 Horgen (CH); DOW BENELUX N.V., 4542 NM Hoek (NL)
(72) Inventor: Delcour, Kees, 4542 BB Hoek (NL); Meertens, Marinus, 4535 GN Terneuzen (NL); Lenoir, Pierre, 8805 Richterswil (CH)
(74) Representative: Huber, Bernhard, Dipl.-Chem.

(57) **Abstract**

A composition mixture having the following general formula (I) below

R₁R₂R₃N (I)

wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)x; or R₁₂-(OCHR₁₃-CH₂)ₚ-; in which
R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6 and protonated and quaternized forms thereof and the processes for the preparation of the same are disclosed.

The composition mixture of formula I above and protonated and quaternized forms thereof can be used in paper and fabric softening products, softergent products, personal care, and lubricant products.

## Description

The present invention concerns new compositions which can be useful in particular in fabric softening applications and a process for the preparation thereof. In particular, the present invention relates to alkoxylated ester-amine fabric softening compositions, the protonated or quaternized forms thereof, and processes for the preparation of the same.

Compositions suitable for providing fabric softening and static control benefits during laundering are well known and have found wide commercial application. Conventionally, rinse-added fabric softening compositions contain as the active softening component, substantially water-insoluble cationic materials having more than one long alkyl chain. Typical of such cationic materials ditallow dimethyl ammonium chloride and immidazolinium compounds substituted with two stearyl groups. These materials are normally prepared in the form of a dispersion in water.

Various quaternized ester-amines (esterquats) are also used as cationic fabric softening agents. See, for example, International Patent Application Publication No. WO 93/25648 (The Procter & Gamble), German Patent No. 4,339,643 (Henkel KGaA)and U.S. Patent Nos. 4,339,391 (Hoffmann et al.); 4,767,547 (Straathof/Konig), 4,874,554 (Lange et al.)and 5,066,414 (Chang).

European Patent Application No. 0'638'639 A1 (Akzo N.V.) teaches various reverse esterquats which are said to be good fabric softening compositions having improved hydrolytic stability and biodegradability.

International Patent Application Publication No. WO 92/08837 (Akzo N.V.) teaches that protonated ester-amines such as protonated 1-dimethylamino-2,3-propanediol (DMAPD) diester in combination with citric acid is a good biodegradable fabric softening agent, although ester-amines are generally believed to be normally poor softeners. However, we found these softening agents not to be hydrolytically stable.

International Patent Application Publication Nos. WO 94/04641 and WO 94/04642 (Colgate Palmolive Company) describe a biodegradable softeners comprising an alkanolamine triester and/or diester in combination with an organic or mineral acid. However, these softening compositions are not hydrolytically stable.

Surprisingly, we have discovered that alkoxylated ester-amines of formula I below and their protonated and quaternized forms of formula II and III, respectively below are not only good softeners but are also hydrolytically stable and, thus, they can be used to formulate stable softener formulations. Since the alkoxylated ester-amines of the present invention are often low viscous liquids at room temperature, they can be advantageously mixed with water and acids at room temperature.

Various alkoxylated quats are used as cationic fabric softening agents. See, for example, U.S. Patent No. 4,844,822 (Fox et al.) European Published Patent Application No. 0'182'669 (Atlantic Richfield Company).

More recently, alkoxylated esterquats prepared by first esterifying an alkoxylated alkanolamine and then quaternizing so obtained alkoxylated ester-amine with a suitable quaternizing agent have been suggested as fabric softening agents. See, for example, International Patent Application Publication No. WO 91/01295 (Henkel), German Patent Nos. 4,308,792 and 4,335,782, and German Published Patent Application No. 4,2443,547 A1. The processes described in the above mentioned documents are clearly different from the process of the present invention in that they use already alkoxylated alkanolamine as a starting compound. In contrast, in the process embodiments of the present invention an alkanolamine is used as the starting compound. In one embodiment of the present process an alkanolamine is first alkoxylated, followed by the esterification and quaternization of the respectively produced compounds. In another embodiment of the present process an alkanolamine is first esterified, followed by the alkoxylation of the resulting ester-amine and, if desired, quaternization of the resulting alkoxylated ester-amine. Not only is the process described in the aforementioned prior art different from the processes of the present invention but the alkoxylated ester-amine produced by the respective processes are also clearly different chemical entities.

Although satisfactory results may be obtained with one or more of the above prior art fabric softening compositions, further improvements are desired in terms of hydrolytic stability over wide pH range, low melting properties for ease of formulation, compatibility with detergent and personal care ingredients, and handling properties so that they can be handled as liquids or formulated as stable fabric softener concentrates.

It has now surprisingly been found that softening compositions of the present invention exhibit-high hydrolytic stability over a wide pH range, have low melting points, are compatible with detergent and personal care ingredients and they can be handled either in the diluted form or formulated as stable liquid concentrates.

As used herein, the term "composition" embraces both a single chemical entity and a mixture of chemical entities having the same general formula.

The present invention concerns a composition having the following general formula

R₁R₂R₃N (I)

wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6;

In another embodiment the present invention concerns a composition having the following general formula

R₁R₂R₃N (HA)_{y} (II)

wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ- in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6; A is an inorganic or organic acid anion; and 0 < y ≤ 1.

In another embodiment the present invention concerns a composition having the following general formula

R₁R₂R₃N (QA)_{y} (III)

wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; with the proviso that R₄ and R₅ are not all H or methyl; R₆ is H and R₇-CO-, with the proviso that the ratio of R₇-CO- to H is less than 10; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6; Q is C₁ to C₆ alkyl; or C₆ to C₁₂ aryl optionally substituted with an alkyl; or HO-CHR₄-CH₂-, wherein R₄ is as previously defined; A is an inorganic or organic acid anion; and 0 < y < 1.

Still in another embodiment the present invention concerns a composition having the following general formula

R₁R₂R₃N (I)

or

R₁R₂R₃N (HA)_{y} (II)

or

R₁R₂R₃N (QA)_{y} (III)

or a mixture thereof wherein R₁ is a group having the formula R₇-CO-OCHR₄-CH₂- or CₙH₍₂ₙ₊₁₋ₓ₎(OR₈)ₓ; in which R₄ is H or C₁-C₄ alkyl; R₇ is C₅-C₃₅ linear or branched saturated or unsaturated alkyl; R₈ is independently, in each occurrence, H; or R₇-CO-; or R₆-(OCHR₅-CH₂)_{b}-,in which R₆ is H or R₇-CO-, with the proviso that at least one but not all of R₈ is R₇-CO-; b is 0 to 30; n is 2 to 6; and x is 2 to 6; R₂ is C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 1 to 30; d is 0 to 30, with the proviso that in at least one R₈ d is other than 0; n is 2 to 6; p is 1 to 30; and x is 2 to 6; with the proviso that when R₂ is C₁-C₂₄ alkyl group or the R₁₂-(OCHR₁₃-CH₂)ₚ- group, then R₁ is CₙH₍₂ₙ₊₁₋ₓ₎(OR₈)ₓ in which at least one R₈ is the R₇-CO- group and at least one R₈ is the R₆-(OCHR₅-CH₂)_{b}- group having b different than 0; and R₃ is independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6; Q is C₁ to C₆ alkyl; or C₆ to C₁₂ aryl optionally substituted with an alkyl; or HO-CHR₄-CH₂-, wherein R₄ is as previously defined; A is an inorganic or organic acid anion; and 0 < y ≤ 1.

Preferably, at least one of R₁, R₂ and R₃ in formulae I, II and III above should contain a carbon chain having from 6 to 36 carbon atoms, more preferably a carbon chain having from 9 to 24 carbon atoms.

Compositions of the present invention of formula I, II and III above in which R₇ is C₈ to C₂₃ linear or branched saturated or unsaturated alkyl, a is 1 to 6 and b, c, and d independently are 0 to 6 are preferred compositions.

Compositions of the present invention of formulae I, II an III above in which at least one of R₂ or R₃ is C₁ to C₄ alkyl are further preferred.

Compositions of the present invention of formulae I, II and III above in which R₅ in the (O-CHR₅-CH₂) group attached directly to the -OCHR₄-CH₂- group of the R₆-(O-CHR₅-CH₂)ₐ-OCHR₄-CH₂- group is not H if R₄ is H, and in which R₅ in the CₙH₍₂ₙ₊₁₋ₓ₎(OR₈)ₓ group is not H in the OR₈ group directly attached to the CₙH₍₂ₙ₊₁₋ₓ₎ group are more preferred.

Compositions of the present invention of formulae I, II and III above in which at least one R₅ is C₂ to C₄ alkyl are particularly preferred.

In another aspect the present invention concerns a composition comprising at least one composition of formulae I, II and III above.

Still in another aspect the present invention concerns a composition comprising at least one composition of formulae I, II and III above or a mixture thereof and at least one known fabric softening composition.

Still in another embodiment the present invention concerns a composition comprising at least one composition of formulae I, II and III above or a mixture thereof and at least one known softener, detergent, personal care or lubricant product ingredient.

Yet in another embodiment the present invention concerns a process for the preparation of a composition which process comprises the steps of
(a) alkoxylating a compound having the general formula

   R₉R₁₀R₁₁N (IV)

   wherein
   R₉ is HOCHR₄CH₂-; or CₙH₍₂ₙ₊₁₋ₓ₎(OH)ₓ-;
   R₁₀ and R₁₁ are each independently C₁ to C₂₄ alkyl; or HOCHR₄CH₂-; or R₁₂-(OCHR₁₃-CH₂)ₚ-; or CₙH₍₂ₙ₊₁₋ₓ₎(OH)ₓ-; R₄ is H or C₁-C₄ alkyl; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom;
   n and x are each independently 2 to 6; and
   p is 1 to 30;
      with an C₂ to C₆ alkylene oxide such as, for example, ethylene oxide, propylene oxide and butylene oxide, or mixtures of such oxides (block or random) in the presence of an alkaline catalyst such as sodium, potassium, calcium, barium and strontium hydroxide, in an amount of from 0.01 to 5, preferably 0.1 to 0.5, percent by weight based on the total weight of the mixture at the completion of the reaction. The alkoxylation reaction is carried out at an elevated temperature, preferably at a temperature from 50°C to 200°C, more preferably from 80°C to 120°C and a pressure of from 1 to 80 bars. If desired, the alkoxylation reaction may be carried out in the presence of a suitable inert solvent;
(b) esterifying at least a portion of the alkoxylated amine product obtained in step (a) with a fatty acid or a mixture of fatty acids at an elevated temperature, preferably at a temperature from 50°C to 250°C, more preferably from 180°C to 220°C and reduced pressure, preferably from 1 to 500 mbar, more preferably from 20 to 200 mbar, pressure. The esterification reaction is carried out until the residual content of said fatty acid is less than 30 percent by weight of the total weight of the reaction mixture; and
(c) reacting at least a portion of the alkoxylated ester-amine compound obtained in step (b) with at least one compound of the formula HA or QA, wherein Q is C₁ to C₆ alkyl; or C₆ to C₁₂ aryl optionally substituted with an alkyl; or HO-CHR₄-CH₂-; R₄ is H or C₁-C₄ alkyl; and A is an inorganic or organic acid anion; at the molar ratio of HA/alkoxylated ester-amine compound of from 0.05 to 2, or the molar ratio of QA/alkoxylated ester-amine compound of from 0.1 to 20, and at a temperature of from 30°C to 150°C, preferably from 75°C to 95°C and a pressure of from 1 to 50 bars in case of QA, or at a temperature of from room temperature to 100°C in case of HA; If desired, the reaction in this step (c) may be carried out in the presence of at least one additive such as water, isopropanol, propanediol, dipropylene glycol, PEG, PPG, alkoxylated fatty acids and alcohols having more than 7 carbons in the fatty chain, glycol ether solvents such as Dowanol® P and E series, diether solvents such as Proglyde® DMM, tetrahydrofuran, methanol, ethanol, hexanediol, acetone and the like which depresses the melting point of the reaction mixture. The additives can be added at any stage during the step (c).

Yet in another embodiment the present invention concerns a process for the preparation of a composition comprising the steps of
(a) esterifying a compound having the general formula

   R₉R₁₀R₁₁N (IV)

   wherein R₉ is HOCHR₄CH₂-; or CₙH₍₂ₙ₊₁₋ₓ₎(OH)ₓ-; R₁₀ and R₁₁ are each independently C₁ to C₂₄ alkyl; or HOCHR₄CH₂-; or R₁₂-(OCHR₁₃-CH₂)ₚ-; or CₙH₍₂ₙ₊₁₋ₓ₎(OH)ₓ-; R₄ is H or C₁-C₄ alkyl; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; n and x are each independently 1 to 6; and p is 1 to 30;
   with a fatty acid or mixture of fatty acids at an elevated temperature, preferably at a temperature from 50°C to 250°C, more preferably from 180°C to 220°C and reduced pressure, preferably from 1 to 500 mbar, more preferably from 20 to 200 mbar, pressure. The esterification reaction is carried out until the residual content of said fatty acid is less than 30 percent by weight of the total weight of the reaction mixture and so that 5 to 90 percent of the available OH groups remain unreacted; and
(b) alkoxylating at least a portion of the ester amine compound obtained in step (a) with an C₂ to C₆ alkylene oxide such as, for example, ethylene oxide, propylene oxide and butylene oxide or mixtures of such oxides (block or random) in the presence of an alkaline catalyst such as sodium, potassium, calcium, barium and strontium hydroxide, in an amount of from 0.01 to 5, preferably 0.1 to 0.5, percent by weight based on the total weight of the mixture at the completion of the reaction. The reaction is carried out at an elevated temperature, preferably at a temperature from 50°C to 200°C, more preferably from 80°C to 120°C and a pressure of from 1 to 80 bars. If desired, the alkoxylation reaction may be carried out in the presence of a suitable inert solvent. Optionally, at least a portion of the alkoxylated ester-amine product obtained in step (b) is further reacted with at least one compound of the formula HA or QA, wherein Q is C₁ to C₆ alkyl; or C₆ to C₁₂ aryl optionally substituted with an alkyl; or HO-CHR₄-CH₂-; R₄ is H or C₁-C₄ alkyl; and A is an inorganic or organic acid anion; at the molar ratio of HA/alkoxylated ester-amine compound of from 0.05 to 2, or the molar ratio of QA/alkoxylated ester-amine compound of from 0.1 to 20, and at a temperature of from 30°C to 150°C, preferably from 75°C to 95°C and a pressure of from 1 to 50 bars in case of QA, or at a temperature of from room temperature to 100°C in case of HA; If desired, this reaction may be carried out in the presence of at least one additive such as water, isopropanol, propanediol, dipropylene glycol, PEG, PPG, alkoxylated fatty acids and alcohols having more than 7 carbons in the fatty chain, glycol ether solvents such as Dowanol® P and E series, diether solvents such as Proglyde® DMM, tetrahydrofuran, methanol, ethanol, hexanediol, acetone and the like which depresses the melting point of the reaction mixture. The additives can be added at any stage after step (b).

The alkoxylation reaction in the aforementioned processes may be carried out in the presence of an alkaline catalyst, such as sodium, potassium, calcium, barium and strontium hydroxide, in an amount of from 0.01 to 5, preferably 0.1 to 0.5, percent by weight based on the total weight of the mixture at the completion of the reaction. Temperature and pressures are not critical, but conveniently the alkoxylation reaction is carried out at an elevated temperature, preferably at a temperature from 50°C to 200°C, more preferably from 80°C to 120°C and a pressure of from 1 to 80 bars. The alkaline catalysts suitable for use in this reaction are well known to a person skilled in the art. After completion of the reaction, that is, for example, when the pressure does not change anymore, the catalyst is removed by a suitable method, such as by filtration over an absorbing clay, for example, magnesium silicate, or neutralized with an inorganic acid such as, for example, hydrochloric acid, or an organic acid such as, for example, acetic acid. If desired, an excess of an acid can be used, so that the excess of the acid can serve as a catalyst in the subsequent reaction step. It is advantageous to carry out the alkoxylation reaction in the presence of a defoaming agent.

The esterification reaction in the aforementioned processes is carried out with a fatty acid or a mixture of fatty acids. The esterification reaction is carried out at an elevated temperature, preferably at a temperature from 50°C to 250°C, more preferably from 180°C to 220°C, and reduced pressure, preferably from 1 to 500 mbar, more preferably from 20 to 200 mbar.

Suitable examples of fatty acids useful in the esterification reaction include valeric acid, caproic acid, caprylic acid, capric acid, lauric acid, myristic acid, palmitic acid, stearic acid, arachidic acid, behenic acid, and lignoceric acid, and the branched isomers thereof (isovaleric acid, for example), or the unsaturated isomers thereof (for example, oleic acid). Of course, these fatty acids are well-known to the person of ordinary skill in the art, and need no further explanation here.

The alkoxylated ester-amine compounds obtained in the aforementioned processes can further be neutralized with a compound of the formula HA at the molar ratio of HA/alkoxylated ester-amine compound of from 0.05 to 2 at room temperature or above the melting point of the alkoxylated ester-amine compound, or quaternized with the compound of the formula QA at the molar ratio of QA/alkoxylated ester-amine compound of from 0.1 to 20, at the temperature of from 30°C to 150°C, and a pressure of from 1 to 50 bars. Any known inorganic acid such as, for example, hydrochloric acid, or organic acid such as, for example, citric acid can be used as the HA compound to protonate the alkoxylated ester-amine (see for example, International Published Patent Application Nos. WO 94/04641 and WO 94/04642). The protonation reaction is suitably carried out at room temperature or above melting point of the alkoxylated ester-amine compound. Any known quaternizing agent can be used as the QA compound. Suitable quaternizing agents of formula QA include alkyl halides, dialkyl sulfates, and trialkyl phosphates. Preferred alkyl halides include methyl chloride, ethyl chloride, methyl bromide, and ethyl bromide; preferred dialkyl sulfates include dimethyl sulfate, and diethyl sulfate, and preferred trialkyl phosphates include trimethyl phosphate and triethyl phosphate. The quaternization reaction is suitably carried out at a temperature of from 30°C to 150°C, and a pressure of from 1 to 50 bars. It is advantageous to carry out the quaternization reaction in the presence of a defoaming agent.

The compositions of formulae I, II and III of the present invention exhibit good softening, antifoaming and antistatic properties, they have low melting points, are hydrolytically stable at various pH values. Moreover, they are compatible even with fabric and paper softeners' ingredients and detergents ingredients which are normally not compatible with known softening compounds without the presence of special additives. Due to these properties the composition mixtures of formulae I, II and III of the present invention can be formulated into formulations suitable for various end use applications such as, for example, fabric and paper softening formulations, formulation for cleaning and conditioning fabric materials ("softergents"), antifoaming formulations, personal care formulations, lubricating formulations, drilling fluid formulations and hard surface cleaning formulations.

Thus, in another aspect the present invention concerns a fabric or paper softening formulation comprising at least one composition of formulae I, II and III above or a mixture thereof and, optionally, at least one known softening compound. The fabric and paper softening formulations of the present invention can also incorporate on or more known ingredients commonly used in fabric or paper softening formulations.

The fabric and paper softening formulations of the present invention can be in any form such as for example, aqueous or anhydrous liquid formulations, super concentrate liquid formulations, solid formulations obtained by a suitable process such as grinding the softener composition or depositing it onto solid substrates such as powders or granules, or onto cellulosic substrate sheets for use in tumble dryers. The fabric softening formulations can be used in a tumbler dryer or in the rinse cycle in a washing machine for example by dispensing the fabric softening formulation via a dispensing compartment optionally with dilution prior to dosing into the dispensing compartment. The fabric and paper softening formulations of the present invention which have pH below 8 when diluted for normal use conditions are preferred.

Thus in another embodiment the present invention concerns an anhydrous or aqueous fabric and paper softening formulation comprising at least 0.01, preferably from 0.01 to 95 percent by weight of at least one composition of the above general formulae I, II and III.

The liquid fabric and paper softening formulations of the present invention may be prepared by mixing at least one softening composition of formulae I, II and III above with a liquid carrier and, optionally, at least one other above mentioned ingredient in a standard formulation mixing equipment and in accordance with techniques known to a person skilled in the art. Low-shear mixing is generally sufficient to adequately and uniformly mix the softening composition within the softening formulation. The final softening formulation, whether in concentrated or diluted form must be easily pourable by the end user. For concentrated softening formulation, which can be used without any dilution if desired, the formulation may be formulated to be diluted (for example for refill packs) by a factor of generally 4:1 or more, preferably up to 8:1 or even up to 20:1.

Commercial fabric softener formulations contain normally from 5 to 20 percent of softening agent. They are normally opaque milky emulsions of various viscosities. In general the viscosity of fabric softening formulations increase with the increase in concentration of its fabric softening agent. Additives are normally used to stabilize more concentrated fabric softening formulations (for example those containing between 10 and 20 percent softening agent). It should be noted that International Published Patent Application No. WO 96/21715 (Procter and Gamble) teaches the use of special deflocculating nonionic polymeric surfactants to allow incorporation of up to 35 percent of a softening agent. The absence of additives would lead to gelling of softening formulations containing more than 15 percent of a softener agent.

It has now surprisingly been discovered that not only compositions of the present invention of formulae I, II and III above have unexpectedly higher hydrolytic stability than known softening agents but they also have lower melting points than known softening agents. Without desire to be bound to a theory, it is believed that this unique combination of high hydrolytic stability and low melting points of compositions of formulae I, II and III above allow for the formulation of stable super-concentrated and, if desired, anhydrous fabric softening formulations with or without the incorporation of any additives. Even more surprisingly, it has been discovered that super concentrated and anhydrous fabric softening formulations incorporating at least one composition of formulae I, II and III above are transparent in most instances.

The present invention also encompasses the super concentrated fabric softening formulations comprising from 10 to 100 percent of at least one composition of formulae I, II and III above. These super concentrated formulations, whether transparent or not, can be used for example for a refill pack (to be diluted in a large bottle) or as concentrates for direct use by a consumer, or can help save transportation costs of the softener or the fabric or paper softening formulations .

Mixtures of fabric softeners and detergents (softergents) are commercially available. Such formulations can be in liquid, paste or granular form. Such softergents normally contain soft clays and/or cationic surfactants having only one long alkyl in the molecule (for example, lauryltrimethyl ammonium chloride). It is known that the known softergents have a number of limitations. For example, the softening performance of these softergents is inferior to the performance of a fabric softener used separately in the last rinse of the wash process. Another limitation is the difficulty in formulating softergents containing cationic softener having more than one long alkyl chain in the molecule (common softening agent used in softening formulations). Still another limitation is the fact that a cationic softener having one or more ester links must be formulated at low pH which does not correspond to typical neutral to alkaline pH of the detergent.

It has now surprisingly been discovered that due to the compatibility of compositions of the present invention of formulae I, II and III above with conventional detergent ingredients and because they are hydrolytically stable at typical detergent pH's they can conveniently be formulated with any known detergent ingredients into a softergent having none of the aforementioned limitations.

Thus, in another aspect the present invention concerns a powder or liquid softergent formulation comprising 1 to 90 weight percent of at least one composition of formulae I, II and III above, which softergent formulation has pH in the range of from 1.5 to 12. In case of powder softergent, the absence of solvent in the composition of formulae I, II and III above makes the softergent safer to use. Moreover, the composition of formulae I, II and III above permits the selection of a softener which has low enough melting point to be suitable for making powder softergent and allows optimum dispersion during the wash cycle.

It was also surprising to discover that compositions of the present invention of formulae I, II and III perform well as foam controlling agents.

Thus, in another aspect the present invention concerns defoaming or antifoaming aqueous formulations comprising at least one composition of formulae I, II or III above. The defoaming and antifoaming aqueous formulations of the present invention are suitable for use in applications such as sugar beet washing, dishwashing and the like, at the pH range of from 1.5 to 12, although due to their improved hydrolytic stability they are particularly suitable for use in the mid-alkaline pH range.

The compositions of the present invention of formulae I, II and III above can suitably be formulated with a wide variety of known materials commonly found in fabric or paper softening formulations, laundry detergents, hard surface cleaning formulations and personal care formulations. Such materials used in fabric or paper softening formulations, laundry detergents, hard surface cleaning formulations and personal care formulations include, but are not limited to the following.
(a) Enzymes and Enzyme Stabilizers - Enzymes can be included for various fabric cleaning and fabric softening purposes. Nonlimiting examples of suitable enzymes include proteases, amylases, lipases, cellulases, and peroxidases, as well as mixtures thereof. The enzymes may be of any suitable origin, such as vegetable, animal, bactericidal, fungal and yeast origin. The enzymes used may be stabilized by the presence of water-soluble sources of calcium and/or magnesium ions in the finished compositions which provide such ions. Any water-soluble calcium or magnesium salt can be used as a source of calcium or magnesium ions. A wide range of useful enzyme and enzyme stabilizer materials are described in International Patent Application Publication Nos. WO 95/19951 and WO 96/21715, and European Patent Application Nos. 0'579'295 A1 and 0'583'536 A1, all to The Procter & Gamble Company, and references sited therein.
(b) Bleaching Agents and Bleach Activators - Any known bleaching agent used in fabric or paper treatment applications can be used. Nonlimiting examples of suitable bleaching agents include oxygenated bleaches, percarboxylic acid bleaches, peroxygen bleaches and mixtures thereof. Bleach activator can also be used. Various nonlimiting examples of useful bleaching agents and bleach activators are given in International Patent Application Publication No. WO 95/19951 (The Procter & Gamble Company) and references cited therein.
(c) Builders - Inorganic and organic builders commonly used in fabric laundering formulations to assist in removal of particulate solids can be used. Suitable builders include, but are not limited to, phosphates, polyphosphates, silicates, aluminosilicates, phosphonates, carboxylates, zeolites and succinates. Nonlimiting examples of suitable builders are described in International Patent Application Publication No. WO 95/19951 and European Patent Application No. 0'579'295 A1, both to The Procter & Gamble Company, and European Patent Application No. 0'580'245 A2 (Colgate-Palmolive Company), and references cited therein.
(d) Soil Release Agents - Any known polymeric soil release agent used in laundry cleaning formulations can be used. Polymeric soil release agents include, but are not limited to, the compounds having: (i) at least one nonionic hydrophylic component consisting essentially of (a) polyoxyethylene segments with a degree of polymerization of at least 2, or (b) oxypropylene or polyoxypropylene segments with a degree of polymerization of from 2 to 10., or (c) a mixture of oxyalkylene units comprising oxyethylene and from 1 to 30 oxypropylene units, (d) cellulosic derivatives such as hydroxyether cellulosic polymers, (e) copolymeric blocks of therephthalate with polyethylene oxide or polypropylene oxide. Nonlimiting examples of useful soil release agents are given in International Patent Application Publication No. WO 95/04802, WO 93/23510 and WO 93/25648, all to Procter & Gamble, and references cited therein.
(e) Chelating agents - Any known chelating agent is suitable for use. Suitable chelating agents include, but are not limited to, amino carboxylates, amino phosphonates, polyfunctionally-substituted aromatic chelating agents and mixtures thereof. It is believed that the benefit of the chelating materials is due in part to their exceptional ability to remove iron and manganese ions from washing solutions by formation of soluble chelates. Nonlimiting examples of suitable chelating agents are described in International Patent Application Publication Nos. WO 95/19951 and WO 96/21715, both to The Procter & Gamble Company, and references cited therein.
(f) Clay Soil Removal/Anti-Redeposition Agents - Any water-soluble alkoxylated amines having clay soil removal and anti-redeposition properties normally used in granular or liquid detergents can be used. Nonlimiting examples of useful clay soil removal/anti-redeposition agents are described in International Patent Application Publication No. WO 95/19951 (The Procter & Gamble Company) and references cited therein.
(g) Dispersing Agents - Suitable dispersing agents are polymeric dispersing agents such as, for example, polymeric polycarboxylates and polyethylene glycols, normally used in detergents. Nonlimiting examples of the dispersing agents are given in International Patent Application Publication No. WO 95/19951 (The Procter & Gamble Company) and references cited therein. Protonated amines, such as those described in International Patent Application Publication No. WO 93/25648 (The Procter & Gamble Company) and references cited therein, and terephthalate/alkylene oxide copolymers, such as those described in International Patent Application Publication No. WO 96/21715 (The Procter & Gamble Company) and references cited therein can be used to enhance dispersion stability.
(h) Optical Brighteners - Any known brightener used in detergents can be used. Suitable brighteners include, but are not limited to, derivatives of stilbene, pyrazoline, coumarin, and carboxylic acid. Nonlimiting examples of suitable brighteners are given in International Patent Application Publication Nos. WO 95/19951 and WO 96/21715, both to Procter & Gamble Company, and references cited therein.
(i) Suds Suppressors - Any known compound that suppresses or reduces the formation of suds is suitable for use. Such compounds include, but are not limited to, silicones, silica-silicone mixtures, monocarboxylic fatty acids and soluble salts thereof, high molecular weight hydrocarbons such as paraffin, and fatty acid esters of monoalcohols. These and other suitable suds suppressors are described in International Patent Application Publication No. WO 95/19951 and European Patent Application No. 0'579'295 A1, both the Procter & Gamble Company, and references cited therein.
(j) Fabric Softeners - Any known fabric softener compound can be used. Nonlimiting examples of suitable fabric softener compounds include clay softeners, conventional quaternary ammonium softening agents, anionic softeners, nonionic softeners, and cationic softeners. These and other suitable fabric softeners are described in International Patent Application Publication Nos. Wo 95/04802, WO 95/19951, and WO 96/21715, all to The Procter & Gamble Company, and European Patent Application No. 0'580'245 A2 (Colgate-Palmolive Company, and references cited therein.
(k) Detersive Surfactants - Various surfactant materials such as anionic, nonionic, cationic, ampholytic, and zwitterionic surfactants can be used. Nonlimiting examples of suitable surfactant include alkyl sulfonates, C₁₁-C₁₈ alkyl benzene sulfonates ("LAS"), primary and secondary branched -chain and random C₁₀-C₂₀ alkyl sulfates ("AS"), and polyhydroxy fatty acid amide surfactants. These and other suitable surfactants are described in International Patent Application Publication Nos. WO 93/23510, WO 25648 and WO 95/19951, all to The Procter & Gamble Company, European Patent Application Nos. 0'579'296 A1 and 0'583'536 A1, both to The Procter & Gamble Company, and European Patent Application No. 0'580'245 A2 (Colgate-Palmolive Company), and references cited therein.

Other materials which can optionally be included are liquid carriers such as, for example, water and C₁ to C₄ monohydric alcohols, thickening agents, viscosity control agents, di-(higher alkyl) cyclic amines, aqueous emulsions of predominantly linear polydialkyl or alkyaryl siloxanes absorbency enhancers, pH modifiers such as bases and acids, nonionic or other deflocculating agents, hydrotropes, colorants, perfumes, perfume carriers, preservatives, opacifiers, fluorescers, anti-shrinking agents, anti-wrinkle agents, anti-spotting agents, bactericides, germicides, fungicides, anti corrosion agents, drape imparting agents, antistatic agents, ironing agents, wetting agents, strength additives such as caboxymethyl cellulose and water-soluble cationic polymers, and the like. These optional materials are well known and widely used in the art. See, for example, International Patent Application Publication Nos. WO 95/19951, WO 93/25648, WO 93/23510, WO 96/21715, WO 96/09436 and WO 94/29521, all to The Procter & Gamble Company, and European Patent Application Nos. 0'580'245 A2 (Colgate-Palmolive Company), and references cited therein.

Various processes for formulating active ingredients with additional materials into formulations useful in fabric or paper softening applications, laundry detergent applications, hard surface cleaning applications and personal care applications are known and widely used in the industry. Some of the processes are described in the references cited herein before.

The following designations, symbols, terms and abbreviations are used in the examples which follow:
- BO: is butylene oxide
- EO: is ethylene oxide
- PO: is propylene oxide
- TEA: is triethanolamine
- MDEA: is methyldiethanolamine
- DMAPD: is 1-(dimethylamino)-2,3-propanediol
- FA₁₈: is stearic acid
- FA₄₀₆: a fatty acid sold by FINA Chemicals under the trademark Radiacid® 406. It is mostly C₁₆-C₁₈ based fatty acids having some degree of unsaturation.

The following examples are given to illustrate the invention and should not be interpreted as limiting it in any way. Unless stated otherwise, all parts and percentages are given by weight.

### EXAMPLE 1

### (a) Alkoxylation Step

Propylene oxide was fed to a reaction vessel containing triethanolamine (propylene oxide/triethanolamine mole ratio of 3:1) and potassium hydroxide catalyst (0.5 percent by weight based on the weight of the product obtained) whilst the temperature of the reaction vessel was kept at 100 to 105°C. Potassium hydroxide was added to triethanolamine as a 45 percent by weight aqueous solution, after which the reaction mixture was kept at 100°C and pressure of about 20 mbar for one additional hour to remove water which is formed in the reaction of potassium hydroxide with TEA. The temperature of the exothermic reaction of PO with TEA was controlled by cooling the reaction vessel and controlling the rate of addition of propylene oxide. After the addition of propylene oxide has been completed, the reaction was allowed to continue at 120°C until the pressure in the reaction vessel became constant (usually after 2 to 3 hours). The potassium hydroxide catalyst was then removed by stirring the reaction mixture with tenfold amount of magnesium silicate for several minutes at 60°C then filtering the mixture. The product obtained is propoxylated triethanolamine

### (b) Esterification Step

The propoxylated triethanolamine product obtained in step (a) above and Radiacid® 406 fatty acid were mixed in 1:2 mole ratio in a vessel equipped with stirrer and a Dean-Stark set-up and the reaction vessel was gradually heated to 200°C at a pressure of 200 mbar in about 1 to 3 hours. The reaction mixture was kept at this temperature and pressure for another 1 to 3 hours during which 90 to 95 percent of water which formed was distilled off. The pressure was then reduced to 20 mbar and the heating continued for 20 hours, after which the residual acid content was 1.9 percent by weight. The product obtained (liquid at room temperature) is an ester of propoxylated triethanolamine.

### (c) Quaternization Step

A one liter low pressure autoclave was charged with the propoxylated ester-amine product obtained in step (b) above and the temperature was raised to 95°C. Methyl chloride (1.025 mole/mole) was then introduced into the reaction vessel. The reaction was allowed to proceed for 24 hours. Then, the reactor was degassed and the liquid product was discharged. The 75 percent quaternized propoxylated ester-amine product thus obtained is designated TEA-3PO-2FA₄₀₆-Q. This product is liquid at 35°C and less than 10 percent of the product hydrolyzed after four weeks at 50°C from a 5 percent dispersion in water at pH 4.

### EXAMPLE 2

### (a) Esterification Step

Triethanolamine and stearic acid were mixed in 1:2 mole ratio in a vessel equipped with stirrer and a Dean-Stark set-up and the reaction vessel was gradually heated to 200°C at the pressure of 200 mbar in about 1 to 3 hours. The reaction mixture was kept at this temperature and pressure for another 1 to 3 hours during which 90 to 95 percent of water which formed was distilled off. The pressure was then reduced to 20 mbar and the heating continued for 4 hours, after which the residual acid content is 0.55 percent by weight. The product obtained is an ester of triethanolamine (ester-amine). This product melts at about 40°C.

### (b) Alkoxylation Step

Propylene oxide was fed to a reaction vessel containing the ester-amine product obtained in step (a) above (propylene oxide/ester-amine mole ratio of 3:1) and potassium hydroxide catalyst (0.5 percent by weight based on the weight of the product obtained) whilst the temperature of the reaction vessel was kept at 100 to 105°C. Potassium hydroxide was added to triethanolamine as a 45 percent by weight aqueous solution after which the reaction mixture was kept at 100°C and pressure of about 20 mbar for one hour to remove water which is formed in the reaction of potassium hydroxide with ester-amine. The temperature of the exothermic reaction between propylene oxide and the ester-amine was controlled by cooling the reaction vessel and controlling the rate of addition of propylene oxide. After the addition of propylene oxide has been completed, the reaction was allowed to continue at 105°C until the pressure in the reaction vessel became constant (usually after 20 hours). The potassium hydroxide catalyst was then removed by stirring the reaction mixture with tenfold amount of magnesium silicate for several minutes at 60°C then filtering the mixture. The product obtained is a propoxylated ester-amine which melts at about 40°C.

### (c) Quaternization Step

A one liter low pressure autoclave was charged with the propoxylated ester-amine product obtained in step (b) above and the temperature was raised to 95°C. Methyl chloride (Mole ratio of 1.025) was then introduced into the reaction vessel. The reaction was allowed to proceed for 70 hours. Then, the reactor was degassed and the liquid product was discharged. The 82 percent quaternized propoxylated ester-amine product thus obtained is designated TEA-2FA₁₈-3PO-Q. This product has a melting range of 43 to 46°C. Less than 10 percent of the product hydrolyzed after four weeks at 50°C from a 5 percent dispersion in water at pH 4.

### EXAMPLE 3

### (a) Esterification Step

The process of Example 2, step (a) was repeated except that Radiacid® 406 fatty acid was used instead of stearic acid. The product obtained was an ester of triethanolamine (ester-amine).

### (b) Alkoxylation Step

(i) The process of Example 2, step (b) was repeated except that the product obtained in step (a) above was used instead of the one obtained in step (a) of Example 2 and ethylene oxide (ester-amine/EO mole ratio of 1:1) was used instead of propylene oxide and potassium hydroxide was not removed after completion of the reaction. The product obtained is an ethoxylated ester-amine.
(ii) Then the ethoxylated ester-amine product obtained in step(b)(i) above was further alkoxylated using propylene oxide (ethoxylated ester-amine/PO mole ratio of 1:2.5) by repeating the process of Example 2, step (b). The product thus obtained is an ethoxylated propoxylated ester-amine which is liquid at room temperature.

### (c) Quaternization Step

The process of Example 2, step (c) was repeated except that the ethoxylated propoxylated ester-amine product obtained in step (b) above was used instead of the propoxylated ester-amine product obtained in step (b)of Example 2. The 75 percent quaternized ethoxylated propoxylated ester-amine product thus obtained is designated TEA-2FA₄₀₆-1EO-2.5PO-Q. This product is liquid at 35°C and less than 10 percent of the product hydrolyzed after four weeks at 50°C from a 5 percent dispersion in water at pH 4.

### EXAMPLE 4

The process of Example 3 was repeated except that in step (b)(i) propylene oxide was used instead of ethylene oxide, and in step (b)(ii) ethylene oxide was used instead of propylene oxide. The 85 percent quaternized propoxylated ethoxylated ester-amine product thus obtained is designated TEA-2FA₄₀₆-1PO-2.5EO-Q. This product is liquid at 35°C and less than 10 percent of the product hydrolyzed after four weeks at 50°C from a 5 percent dispersion in water at pH 4.

### EXAMPLE 5

The process of Example 1 was repeated except that in step (a) butylene oxide was used instead of propylene oxide. The 88 percent quaternized butoxylated ester-amine product thus obtained is designated TEA-3BO-2FA₄₀₆-Q. This product is liquid at 35°C and less than 10 percent of the product hydrolyzed after four weeks at 50°C from a 5 percent dispersion in water at pH 4.

### EXAMPLE 6

The process of Example 2 was repeated except that in step (a) Radiacid® 406 fatty acid was used instead of stearic acid and in step (b) butylene oxide was used instead of propylene oxide. The 82 percent quaternized butoxylated ester-amine product thus obtained is designated TEA-2FA₄₀₆-3BO-Q. This product is liquid at 35°C and less than 10 percent of the product hydrolyzed after four weeks at 50°C from a 5 percent dispersion in water at pH 4.

### EXAMPLE 7

The process of Example 1 was repeated except that in step (a) butylene oxide was used instead of propylene oxide and in step (b) Radiacid® 406 fatty acid was used at a mole ratio of BO/Radiacid® 406 fatty acid of 1:2.5). The obtained 64 percent quaternized butoxylated ester-amine product is designated TEA-3BO-2.5FA₄₀₆-Q. This product is liquid at 35°C and less than 10 percent of the product hydrolyzed after four weeks at 50°C from a 5 percent dispersion in water at pH 4.

### EXAMPLE 8

### (a) Alkoxylation Step

(i) The process of Example 1, step (a) was repeated except that butylene oxide was used instead of propylene oxide and the potassium hydroxide catalyst was not removed after completion of the reaction. The product thus obtained is butoxylated triethanolamine.
(ii) Then the butoxylated triethanolamine obtained in step (a)(i) above was further alkoxylated using ethylene oxide (butoxylated triethanolamine/EO molar ratio of 1:3) by repeating the process of Example 1, step (a). The product thus obtained is a butoxylated ethoxylated triethanolamine.

### (b) Esterification Step

The process of Example 1, step (b) was repeated except that stearic acid was used instead of Radiacid® 406 fatty acid and the butoxylated ethoxylated amine product obtained in step (a)(ii) above was used instead of the propoxylated amine product used in Example 1, step (b). The product thus obtained is a butoxylated ethoxylated ester-amine which is liquid at room temperature.

### (c) Quaternization Step

The process of Example 1 step (c) was repeated except that the butoxylated ethoxylated ester-amine product was used instead of the propoxylated ester-amine product used in Example 1, step (c). The 82 percent quaternized butoxylated ethoxylated ester-amine product thus obtained is designated TEA-3BO-3EO-2FA₁₈-Q. This product is liquid at 35°C and less than 10 percent of the product hydrolyzed after four weeks at 50°C from a 5 percent dispersion in water at pH 4.

### EXAMPLE 9

The process of Example 1 was repeated except that in step (a) butylene oxide (at a molar ratio of 6:1) was used instead of propylene oxide and in step (b) stearic acid was used instead of Radiacid® 406 fatty acid. The obtained 73 percent quaternized butoxylated ester-amine product is designated TEA-6BO-2FA₁₈-Q. This product melts in the range of 19 to 22°C. One percent of the product hydrolyzed after four weeks at 50°C from a 5 percent dispersion in water at pH 4.

### EXAMPLE 10

The process of Example 1 was repeated except that in step (a) butylene oxide (at a molar ratio of 6:1 was used instead of propylene oxide. The obtained 87 percent quaternized butoxylated ester-amine product is designated TEA-6BO-2FA₄₀₆-Q. This product is liquid at 35°C and less than 10 percent of the product hydrolyzed after four weeks at 50°C from a 5 percent dispersion in water at pH 4.

### EXAMPLE 11

The process of Example 1 was repeated except that in step (a) methyldiethanolamine was used instead of triethanolamine and butylene oxide (MDEA/BO molar ratio of 1:2) was used instead of propylene oxide. The obtained 97 percent quaternized butoxylated ester-amine product is designated MDEA-2BO-2FA₄₀₆-Q. This product is liquid at 35°C and less than 10 percent of the product hydrolyzed after four weeks at 50°C from a 5 percent dispersion in water at pH 4.

### EXAMPLE 12

The process of Example 1 was repeated except that in step (a) 1-(dimethylamino)-2,3-propanediol was used instead of triethanolamine (DMAPD/Radiacid® 406 fatty acid molar ratio of 1:1.9) and butylene oxide (DMAPD/BO molar ratio of 1:2) was used instead of propylene oxide. The obtained 97 percent quaternized butoxylated ester-amine product is designated DMAPD-2BO-2FA₄₀₆-Q. This product is liquid at 35°C and less than 10 percent of the product hydrolyzed after four weeks at 50°C from a 5 percent dispersion in water at pH 4.

### EXAMPLE 13

### (a) Alkoxylation Step

Propylene oxide was fed to a reaction vessel containing triethanolamine (propylene oxide/triethanolamine mole ratio of 3:1) and potassium hydroxide catalyst (0.5 percent by weight based on the weight of the product obtained) whilst the temperature of the reaction vessel was kept at 100 to 105°C. Potassium hydroxide was added to triethanolamine as a 45 percent by weight aqueous solution, after which the reaction mixture was kept at 100°C and pressure of about 20 mbar for one additional hour to remove water which is formed in the reaction of potassium hydroxide with TEA. The temperature of the exothermic reaction of PO with TEA was controlled by cooling the reaction vessel and controlling the rate of addition of propylene oxide. After the addition of propylene oxide has been completed, the reaction was allowed to continue at 120°C until the pressure in the reaction vessel became constant (usually after 2 to 3 hours). The potassium hydroxide catalyst was then removed by stirring the reaction mixture with tenfold amount of magnesium silicate for several minutes at 60°C then filtering the mixture. The product obtained is propoxylated triethanolamine.

### (b) Esterification Step

The propoxylated triethanolamine product obtained in step (a) above and Radiacid® 406 fatty acid were mixed in 1:2 mole ratio in a vessel equipped with stirrer and a Dean-Stark set-up and the reaction vessel was gradually heated to 200°C at a pressure of 200 mbar in about 1 to 3 hours. The reaction mixture was kept at this temperature and pressure for another 1 to 3 hours during which 90 to 95 percent of water which formed was distilled off. The pressure was then reduced to 20 mbar and the heating continued for 20 hours, after which the residual acid content was 1.9 percent by weight. The product obtained (liquid at room temperature) is an ester of propoxylated triethanolamine. This product is designated TEA-3PO-2FA₄₀₆. This product is liquid at room temperature.

The TEA-3PO-2FA₄₀₆ product was neutralized with HCl by dispersing it at a concentration of about 5 percent in water containing 1 mole of HCl per mole of the TEA-3PO-2FA₄₀₆ product. Thus obtained product is designated TEA-3PO-2FA₄₀₆·HCl. This product has a pH of 2.6and less than 20 percent of the product hydrolyzed after four weeks at 50°C.

### EXAMPLE 14

The process of Example 13 was repeated except that in step (a) methyldiethanolamine was used instead of triethanolamine and butylene oxide (MDEA/BO molar ratio of 1:2) was used instead of propylene oxide. The obtained butoxylated ester-amine product is designated MDEA-2BO-2FA₄₀₆. This product is liquid at room temperature.

The MDEA-2BO-2FA₄₀₆ product was neutralized with HCl by dispersing it at a concentration of about 5 percent in water containing 1 mole of HCl per mole of the MDEA-2BO-2FA₄₀₆ product. Thus obtained neutralized product is designated MDEA-2BO-2FA₄₀₆·HCl. This product has a pH of 2.8 and less than 20 percent of the product hydrolyzed after four weeks at 50°C.

### EXAMPLE 15

The process of Example 13 was repeated except that in step (a) 1-(dimethylamino)-2,3-propanediol was used instead of triethanolamine (DMAPD/Radiacid® 406 fatty acid molar ratio of 1:1.9) and butylene oxide (DMAPD/BO molar ratio of 1:2) was used instead of propylene oxide. The obtained butoxylated ester-amine product is designated DMAPD-2BO-2FA₄₀₆. This product is liquid at room temperature.

The DMAPD-2BO-2FA₄₀₆ product was neutralized with HCl by dispersing it at a concentration of about 5 percent in water containing 1 mole of HCl per mole of the DMAPD-2BO-2FA₄₀₆ product. Thus obtained neutralized product is designated DMAPD-2BO-2FA₄₀₆·HCl. This product has a pH of 3.0 and 21 percent of the product hydrolyzed after four weeks at 50°C.

### COMPARATIVE EXAMPLE 1

Triethanolamine, ethylene oxide, stearic acid and methyl chloride were reacted according to the procedure described in International Patent Application Publication No. WO 91/01295 (Henkel) to prepare alkoxylated esterquat compound designated TEA-3EO-2FA₁₈-Q. This product melts at the temperature of from 56 to 62°C and 24 percent of the product hydrolyzed after four weeks at 50°C from a 5 percent dispersion in water at pH 4.

### COMPARATIVE EXAMPLE 2

Triethanolamine, Radiacid® 406 fatty acid and methyl chloride were reacted according to the procedure described in German Patent No. 4,015, 849 (Henkel) to prepare an esterquat compound designated TEA-2FA₄₀₆-Q. This product melts at the temperature of from 45 to 95°C and 38 percent of the product hydrolyzed after four weeks at 50°C from a 5 percent dispersion in water at pH 4.

### COMPARATIVE EXAMPLE 3

1-(Dimethylamino) 2,3- propanediol, stearic acid and methyl chloride were reacted according to the procedure described in British Patent No. 1,567,947 (Unilever) to prepare an esterquat compound designated DMAPD-2FA₁₈-Q. This product melts at the temperature of from 90 to 120°C and 26 percent of the product hydrolyzed after four weeks at 50°C from a 5 percent dispersion in water at pH 4.

### COMPARATIVE EXAMPLE 4

Methyldiethanolamine, Radiacid® 406 fatty acid and methyl chloride were reacted according to the procedure described in U.S. Patent No. 5,523,433 (Witco) to prepare esterquat compound designated MDEA-2FA₄₀₆-Q. This product melts at the temperature of from 45°C to 85°C and 70 percent of the product hydrolyzed after four weeks at 50°C from a 5 percent dispersion in water at pH 4.

### COMPARATIVE EXAMPLE 5

Triethanolamine, and Radiacid® 406 fatty acid were reacted according to the procedure described in German Patent Application No. 4,015,849 (Henkel) to prepare ester-amine compound designated TEA-2FA₄₀₆. This product melts at the temperature of from 30°C to 45°C.

This product was neutralized by dispersing it at concentration of 5 percent by weight in water containing 1 mole of HCl per mole of the ester-amine product. The neutralized ester amine is designated as TEA-2FA₄₀₆.HCl, has a pH of 2.1 and 57 percent of the product hydrolyzed after four weeks at 50°C.

### COMPARATIVE EXAMPLE 6

Methyldiethanolamine and Radiacid® 406 fatty acid were reacted according to the procedure described in U.S. Patent No. 5,523,433 (Witco) to prepare ester-amine compound designated MDEA-2FA₄₀₆. This product melts at the temperature of from 30°C to 45°C.

This product was neutralized by dispersing it at concentration of 5 percent by weight in water containing 1 mole of HCl per mole of the ester-amine product. The neutralized ester amine is designated MDEA-2FA₄₀₆.HCl, has a pH of 2.5 and 80 percent of the product hydrolyzed after four weeks at 50°C.

### COMPARATIVE EXAMPLE 7

1-(Dimethylamino)-2,3-propanediol and stearic acid were reacted according to the procedure described in British Patent No. 1,567,947 (Unilever) to prepare ester-amine compound designated DMAPD-2FA₁₈. This product melts at the temperature of from 30°C to 45°C.

This product was neutralized by dispersing it at concentration of 5 percent by weight in water containing 1 mole of HCl per mole of the ester-amine product. The neutralized ester amine is designated DMAPD-2FA₁₈.HCl, has a pH of 2.4 and 55 percent of the product hydrolyzed after four weeks at 50°C.

### EXAMPLE 16

A 5 percent emulsion was prepared with each of the Dehyquart® AU 36 (sold by Henkel KGaA), TEA-2FA₄₀₆-3BO-Q and DMAPD-2BO-2FA₄₀₆-Q compositions by mixing the respective composition in molten form with water heated to a temperature close to the melting point of the respective composition. Then 1 ml of each emulsion was added to 100 ml of either deionized water, or deionized water buffered at pH 8 or adjusted to pH 10. The pH 8 buffered deionized water solution was made from Tritisol® buffer 8 (sold by Merck) mixed with deionized water according to directions given by the manufacturer. The pH 10 deionized water was made by adjusting deionized water pH by the addition of a 5 percent solution of NaOH. The dispersion were stored at room temperature and observed daily during one month. The observed results are given in Table 1 below.

**Table 1**

| Composition | Deionized Water | Buffered Deionized Water pH 8 | Deionized Water pH 10 |
|---|---|---|---|
| Dehyquart AU 36* | flocculation after several weeks | immmediate flocculation | immediate flocculation |
| TEA-2FA₄₀₆-3BO-Q | stable turbid emulsion | stable turbid emulsion | stable turbid emulsion |
| DMAPD-2BO-2FA₄₀₆-Q | stable turbid emulsion | stable turbid emulsion | stable turbid emulsion |

| | | | |
|---|---|---|---|
| *not an example of the present invention. | | | |

This example clearly demonstrate that the compositions of the present invention are stable at various pH values. The stability at various pH values of the present invention's compositions is important since it allows for the formulation of these compositions into softening formulations at neutral to mid-alkaline pH values which can incorporate pH sensitive ingredients such as enzymes which are normally stable under these conditions. The stable incorporation of enzymes is not feasible in softener formulations based on standard esterquats due to the acidic pH (1.8-3.5) of these formulations.

### EXAMPLE 17

The 5 percent emulsions of the compositions identified in Table 2 below were prepared according to the procedure of Example 16. 2 ml of each of these emulsions were mixed with 18 ml of 0.5 percent sodium linear dodecyl benzene sulfonate (Na-LAS) solution. Na-LAS anionic surfactant commonly used in laundry detergents is quite incompatible with cationic surfactants (due to the formation of insoluble ion-pairs) without the help of solvents, hydrotropes and the like. The pH of the Na-LAS/respective composition solutions was maintained at 7.5 ± 0.2. The results are given in Table 2 below.

**Table 2**

| Composition | Observations |
|---|---|
| Dehyquart® AU 36* | immediate flocculation |
| TEA-2FA₄₀₆-Q* | immediate flocculation |
| MDEA-2FA₄₀₆-Q* | immediate flocculation |
| DMAPD-2FA₁₈-Q* | immediate flocculation |
| MDEA-2BO-2FA₄₀₆-Q | stable, slightly milky, emulsion |

| | |
|---|---|
| *not an example of the present invention. | |

As seen from Table 2, conventional esterquats precipitate when placed in contact with Na-LAS whereas the composition of the present invention does not precipitate. Thus, it should be noted that the compositions of the present invention can be compatible with Na-LAS without the need to add solvents, hydrotropes, and the like. This demonstrates that the compositions of the present invention can be used in powder or liquid laundry detergents to form laundry detergents having both cleaning and softening properties ("softergents") due to their compatibility with detergent ingredients including anionic compounds and surfactants and their good stability at neutral to mid-alkaline pH values. To our knowledge softergents (liquid or powder) containing meaningful levels of cationic softeners having more than one long carbon chain have not been reported.

### EXAMPLE 18

Ariel® Ultra Liquid ("AUL") liquid laundry detergent (sold by The Procter & Gamble Company and bought in the Netherlands in 1996) contains more than 15 percent of anionic surfactants according to the product label. The measured pH value of AUL used was 7.5. The TEA-2FA₄₀₆-3BO-Q softening composition (8 percent) of Example 6 was added to AUL. For ease of the processing, 20 percent of isopropanol was added to the molten softener composition before it was added to AUL. The so prepared softener formulation remained stable transparent liquid even after 5 months storage at room temperature. Moreover, the hydrolytic stability of TEA-2FA₄₀₆-3BO-Q in AUL stored at 35°C during one month is 90 percent.

Likewise, 10 ml of 5 percent emulsions (made according to the procedure cited in Example 16) of softening compositions identified in Table 3 below were added to 10 ml of AUL and their compatibility determined. The results of the observations are given in Table 3 below.

**Table 3**

| Composition | Observation |
|---|---|
| Dehyquart AU® 36* | immediate precipitation |
| MDEA-2FA₄₀₆-Q* | immediate precipitation |
| TEA-3EO-2FA₁₈-Q* | immediate precipitation |
| TEA-2FA₄₀₆-3BO-Q | AUL stays clear |
| DMAPD-2BO-2FA₄₀₆-Q | AUL stays clear |
| TEA-3PO-2FA₄₀₆-Q | AUL stay clear |
| TEA-2FA₁₈-3PO-Q | AUL stays clear |

| | |
|---|---|
| *not an example of the present invention. | |

It is evident from the above Table 3 that the softening compositions of the present invention can be formulated with liquid or powder detergents containing even high levels of anionic surfactants to form stable softergent formulations.

### EXAMPLE 19

Aqueous and anhydrous super concentrated fabric softening formulations were made using the TEA-6BO-2FA₄₀₆-Q softening composition of the present invention. The formulations ingredients and their physical properties are given in Table 4 below.

**Table 4**

| Formulation Ingredient | Formulation 1 | Formulation 2 | Formulation 3 |
|---|---|---|---|
| TEA-6BO-2FA₄₀₆-Q | 93% | 82% | 49% |
| Perfume | 7% | 6% | - |
| IPA | - | - | 8% |
| Citric Acid | - | 6% | - |
| Water | 0% | 6% | 43% |
| pH | neutral | low | neutral |
| Visual Aspect | transparent | transparent | transparent |

### Example 20

The process of Example 1 was repeated with the exception that in step (b) the molar ratio of propoxylated triethanolamine/Radiacid® 406 fatty acid is 1:1. The 90 percent quaternized propoxylated ester-amine product thus obtained is designated TEA-3PO-1FA₄₀₆-Q. This product is liquid at 35°C and 10 percent of the product hydrolyzed after four weeks at 50°C from a 5 percent transparent solution in water at pH 4. Clarity of the solution was obtained without the use of additives. Thus compositions of the present invention can be formulated into transparent aqueous formulations. In some instances additives are necessary to ensure clarity of the solutions. Normally such additives are hydrophilic.

A composition mixture having the following general formula (I) below

R₁R₂R₃N (I)

wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which
R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6 and protonated and quaternized forms thereof and the processes for the preparation of the same are disclosed.

The composition mixture of formula I above and protonated and quaternized forms thereof can be used in paper and fabric softening products, softergent products, personal care, and lubricant products.

## Claims

1. A composition having the following general formula
R₁R₂R₃N (I)
wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which
R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6.

2. A composition having the following general formula
R₁R₂R₃N (HA)_{y} (II)
wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6; A is an inorganic or organic acid anion; and 0 < y ≤ 1.

3. A composition mixture having the following general formula
R₁R₂R₃N (QA)_{y} (III)
wherein R₁ is a group having the formula R₆-(O-CHR₅-CH₂)ₐ-OCHR₄-CH₂-; or CₙH₍₂ₙ₊₁₋ₓ₎(OR₈)ₓ; in which R₄ is H or C₁-C₄ alkyl; R₅ is independently, in each occurrence, H or C₁-C₄ alkyl; with the proviso that R₄ and R₅ are not all H or methyl; R₆ is H and R₇-CO-, with the proviso that the ratio of R₇-CO- to H is less than 10; R₇ is C₅-C₃₅ linear or branched saturated or unsaturated alkyl; R₈ is independently, in each occurrence, H; or R₇-CO-; or R₆-(OCHR₅-CH₂)_{b}-; a is 1 to 30; b is 0 to 30, with the proviso that in at least one R₈ b is other than 0; n is 2 to 6; and x is 2 to 6; R₂ and R₃ are independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6; Q is C₁ to C₆ alkyl; or C₆ to C₁₂ aryl optionally substituted with an alkyl; or HO-CHR₄-CH₂-, wherein R₄ is as previously defined; A is an inorganic or organic acid anion; and 0 < y < 1.

4. A composition having the following general formula
R₁R₂R₃N (I)
or
R₁R₂R₃N (HA)_{y} (II)
or
R₁R₂R₃N (QA)_{y} (III)
or a mixture thereof wherein R₁ is a group having the formula R₇-CO-OCHR₄-CH₂- or CₙH₍₂ₙ₊₁₋ₓ₎(OR₈)ₓ; in which R₄ is H or C₁-C₄ alkyl; R₇ is C₅-C₃₅ linear or branched saturated or unsaturated alkyl; R₈ is independently, in each occurrence, H; or R₇-CO-; or R₆-(OCHR₅-CH₂)_{b}-, in which R₆ is H or R₇-CO-, with the proviso that at least one but not all of R₈ is R₇-CO-; b is 0 to 30; n is 2 to 6; and x is 2 to 6; R₂ is C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 1 to 30; d is 0 to 30, with the proviso that in at least one R₈ d is other than 0; n is 2 to 6; p is 1 to 30; and x is 2 to 6; with the proviso that when R₂ is C₁-C₂₄ alkyl group or the R₁₂-(OCHR₁₃-CH₂)ₚ- group, then R₁ is CₙH₍₂ₙ₊₁₋ₓ₎(OR₈)ₓ in which at least one R₈ is R₇-CO- group and at least one R₈ is R₆-(OCHR₅-CH₂)_{b}- group having b different than 0; and R₃ is independently C₁-C₂₄ alkyl group; or R₆-(O-CHR₅-CH₂)_{c}-OCHR₄-CH₂-; or CₙH₍₂ₙ₊₁₋ₓ₎(OR₈)ₓ; or R₁₂-(OCHR₁₃-CH₂)ₚ-; in which R₄ is H or C₁-C₄ alkyl; R₅ is independently, in each occurrence, H or C₁-C₄ alkyl; R₆ is H or R₇-CO-; R₇ is C₅-C₃₅ linear or branched saturated or unsaturated alkyl; R₈ is independently, in each occurrence, H; or R₇-CO-; or R₆-(OCHR₅-CH₂)_{d}-; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; c is 0 to 30; d is 0 to 30; n is 2 to 6; p is 1 to 30; and x is 2 to 6; Q is C₁ to C₆ alkyl; or C₆ to C₁₂ aryl optionally substituted with an alkyl; or HO-CHR₄-CH₂-, wherein R₄ is as previously defined; A is an inorganic or organic acid anion; and 0 < y ≤ 1.

5. A composition according to any one of Claims 1 to 3 wherein R₇ is C₈ to C₂₃ linear or branched saturated or unsaturated alkyl, a is 1 to 6 and b, c, and d independently are 0 to 6.

6. A composition according to Claim 4 wherein R₇ is C₈ to C₂₃ linear or branched saturated or unsaturated alkyl, a is 1 to 6 and b and d independently are 0 to 6, c in R₂ is 1 to 6 and c in R₃ is 0 to 6.

7. A composition according to any one of Claims 1 to 6 wherein at least one of R₂ and R₃ is C₁ to C₄ alkyl.

8. A composition according to any one of Claims 1 to 7 wherein R₅ in the (O-CHR₅-CH₂) group attached directly to the -OCHR₄-CH₂- group in the R₆-(O-CHR₅-CH₂)ₐ-OCHR₄-CH₂-group is not H if R₄ is H; and wherein R₅ in the CₙH₍₂ₙ₊₁₋ₓ₎(OR₈)ₓ group should not be H in the OR₈ group directly attached to the CₙH₍₂ₙ₊₁₋ₓ₎ group.

9. A composition according to any one of Claims 1 to 8 wherein at least one R₅ is C₂ to C₄ alkyl.

10. A composition comprising at least one composition as claimed in any one of Claims 1 to 9.

11. A composition comprising at least one composition as claimed in any one of Claims 1 to 10 or a mixture thereof and at least one known softener, detergent, personal care or lubricant product ingredient.

12. A composition comprising at least one composition as claimed in any one of Claims 1 to 11 and at least one known fabric softening composition.

13. A process for the preparation of a composition which process comprises the steps of
(a) reacting a compound having the general formula
R₉R₁₀R₁₁N (IV)
wherein R₉ is HOCHR₄CH₂-; or CₙH₍₂ₙ₊₁₋ₓ₎(OH)ₓ-; R₁₀ and R₁₁ are each independently C₁ to C₂₄ alkyl; or HOCHR₄CH₂-; or R₁₂-(OCHR₁₃-CH₂)ₚ-; or CₙH₍₂ₙ₊₁₋ₓ₎(OH)ₓ-; R₄ is H or C₁-C₄ alkyl; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; n and x are each independently 2 to 6; and p is 1 to 30;
with an C₂ to C₆ alkylene oxide or mixtures of such oxides (block or random) in the presence of an alkaline catalyst in an amount of from 0.01 to 5 percent by weight based on the total weight of the mixture at the completion of the reaction;
(b) esterifying at least a portion of the product obtained in step (a) with a fatty acid or a mixture of fatty acids at an elevated temperature and reduced pressure until the residual content of said fatty acid is less than 30 percent by weight of the total weight of the reaction mixture so that from 5 to 90 percent of the available OH groups remain unreacted; and
(c) reacting at least a portion of the alkoxylated ester-amine compound obtained in step (b) with at least one compound of the formula HA or QA, wherein Q is C₁ to C₆ alkyl; or C₆ to C₁₂ aryl optionally substituted with an alkyl; or HO-CHR₄-CH₂-; R₄ is H or C₁-C₄ alkyl; and A is an inorganic or organic acid anion; at the molar ratio of HA/alkoxylated ester-amine compound of from 0.05 to 2 or at the molar ratio of QA/alkoxylated ester-amine compound of from 0.1 to 20, at a temperature of from 30 to 150 °C, and a pressure of from 1 to 50 bars in case of QA.

14. A process according to Claim 13 wherein after completion of the reaction in step (a) the catalyst is removed or neutralized with a stoichiometric amount of an acid.

15. A process according to Claim 14 wherein after completion of the reaction in step (a) the catalyst is neutralized with an excess of an acid and the excess of the acid is used as a catalyst in step (b).

16. A process according to any one of Claims 13 to 15 wherein the reaction in step (c) is carried out in the presence of at least one additive which depresses the melting point of the reaction mixture.

17. A process for the preparation of a composition comprising the steps of
(a) esterifying a compound having the general formula
R₉R₁₀R₁₁N (IV)
wherein R₉ is HOCHR₄CH₂-; or CₙH₍₂ₙ₊₁₋ₓ₎(OH)ₓ-; R₁₀ and R₁₁ are each independently C₁ to C₂₄ alkyl; or HOCHR₄CH₂-; or R₁₂-(OCHR₁₃-CH₂)ₚ-; or CₙH₍₂ₙ₊₁₋ₓ₎(OH)ₓ-; R₄ is H or C₁-C₄ alkyl; R₁₂ is C₁ to C₂₄ alkyl; R₁₃ is independently, in each occurrence, H or C₁ to C₄ alkyl and with the proviso that R₁₃ is not H in the alkoxy group directly attached to the nitrogen atom; n and x are each independently 2 to 6; and p is 1 to 30;
with a fatty acid or mixtures of fatty acids at an elevated temperature and reduced pressure until the residual content of said fatty acid is less than 30 percent by weight of the total weight of the reaction mixture and so that 5 to 90 percent of the available OH groups remain unreacted; and
(b) reacting at least a portion of the ester-amine compound obtained in step (a) with an C₂ to C₆ alkylene oxide or mixtures (block or random) of such oxides in the presence of an alkaline catalyst in an amount of from 0.01 to 5 percent by weight based on the total weight of the mixture at the completion of the reaction.

18. A process according to Claim 17 wherein at least a portion of the alkoxylated ester-amine obtained in step (b) is further reacted with at least one compound of the formula QA, wherein Q is C₁ to C₆ alkyl; or C₆ to C₁₂ aryl optionally substituted with an alkyl; or HO-CHR₄-CH₂-; R₄ is H or C₁-C₄ alkyl; and A is an inorganic or organic acid anion; at the molar ratio of QA/the alkoxylated ester-amine compound of from 0.1 to 20, at a temperature of from 30°C to 150°C, and a pressure of from 1 to 50 bars.

19. A process according to Claim 17 wherein at least a portion of the alkoxylated ester-amine obtained in step (b) is further reacted with at least one compound of the formula HA, wherein A is an inorganic or organic acid anion; at the molar ratio of HA/alkoxylated ester-amine compound of from 0.05 to 2 and at a temperature of from room temperature to 100°C.

20. A process according to any one of Claims 17 to 19 wherein after completion of the reaction in step (b) the catalyst is removed or neutralized with a stoichiometric amount of an acid.

21. A process according to any one of Claims 17 to 19 wherein in step (a) an acid other than a fatty acid is used as a catalyst.

22. A process according to any one of Claims 18 to-21 wherein the protonation or alkylation reaction after step (b) of claim 17 is carried out in the presence of at least one additive which depresses the melting point of the reaction mixture.

23. A process according to Claim 13 or Claim 22 wherein steps (a) and (b) are successively repeated as many times as desired.

24. The use of a composition as claimed in any one of Claims 1 to 12 or a mixture thereof as foam controlling agent in aqueous foam control formulations.

25. The use of a composition according to any one of Claims 1 to 12 or a mixture thereof in a fabric or paper softening applications.

26. The use of a composition according to any one of Claims 1 to 12 or a mixture thereof in a liquid or powder softergent formulation comprising from 1 to 90 percent by weight of at least one composition as claimed in any one of Claims 1 to 12 or a mixture thereof.

27. The use of a composition according to any one of Claims 1 to 12 or a mixture thereof in a liquid softergent formulation having a pH from 1.5 to 12.

28. The use of a composition according to any one of Claims 1 to 12 or a mixture thereof in an anhydrous fabric or paper softening formulation.

29. The use of a composition according to any one of Claims 1 to 12 or a mixture thereof in a fabric or paper softening formulation comprising from 0.01 percent by weight to 95 percent by weight of at least one composition as claimed in any one of Claims 1 to 12 or a mixture thereof.

30. The use of a composition according to any one of Claims 1 to 12 or a mixture thereof in a transparent fabric or paper softening formulation.

31. The use of a composition according to any one of Claims 1 to 12 or a mixture thereof in a fabric or paper softening formulation comprising at least one composition as claimed in Claims 1 to 12 or a mixture thereof and at least one known compound used in fabric or paper softening formulations.

32. The use of a composition as claimed in any one of Claims 1 to 12 or a mixture thereof in a fabric or paper softening formulation which formulation has pH below 8 when diluted for normal use conditions.

33. The use of a composition mixture as claimed in any one of Claims 1 to 12 or a mixture thereof in an aqueous or anhydrous fabric or paper softening formulation comprising at least one composition mixture as claimed in any one of Claims 1 to 12 or a mixture thereof and a nonionic or cationic emulsifier.

34. The use of a composition as claimed in any one of Claims 1 to 12 or a mixture thereof in a dryer softener sheet comprising at least one composition as claimed in any one of Claims 1 to 12 or a mixture thereof deposited on a cellulose substrate.

35. The use of a composition according to any one of Claims 1 to 12 or a mixture thereof in a solid, powder or granular fabric or paper softening formulation comprising from 0.01 percent by weight to 95 percent by weight of at least one composition as claimed in any one of Claims 1 to 12 or a mixture thereof.
